# EUROPEAN PATENT APPLICATION

(11) **EP 3 896 149 A1**
(43) Date of publication of application: **20.10.2021**
(21) Application number: 21169031.8
(22) Date of filing: 16.04.2021
(51) Int. Cl.: C12M 3/00, C12M 1/12, C12M 1/42

(54) **MODULAR MAGNETICALLY DRIVEN BIOREACTOR FOR CELLULAR CULTURES AND BIOMEDICAL APPLICATIONS**

(30) Priority: 16.04.2020 PT 2020116267
(71) Applicant: Fundacion BCMaterial Basque Center for Materials, Applications and Nanostructures, 48940 Leioa (ES); Universidade do Minho, 4704-553 Braga (PT)
(72) Inventor: LANCEROS MENDEZ, SENEN, 48002 BILBAO (ES); FERNANDES CASTRO, NÉLSON JOSÉ, 4765-583 SERZEDELO GMR (PT); GOMES CORREIA, VITOR MANUEL, 4805-631 SANTA LEOCÁDIA BRITEIROS (PT); DE OLIVEIRA RIBEIRO, CLARISSE MARTA, 4805-498 SÂO LOURENÇO SANDE (PT)
(74) Representative: Patentree

(57) **Abstract**

Modular magnetically driven bioreactor for applying stimuli to cell culture scaffolds comprising: at least one cellular culture plate (d1, d2); a first module comprising a support enclosure (a) and a magnetically inert cover (c) arranged above the support enclosure (a) for supporting the cellular culture plate (d1, d2); an actuator module (b1, b2) arranged below the magnetically inert cover (c); wherein said first module is arranged for receiving said actuator module (b1, b2); wherein the actuator (b1, b2) comprises: a fixed platform, also referred as a fixed table, a movable platform, also referred as a movable table; wherein said movable platform, also referred as a movable table, comprises a plurality of magnets for applying a movable magnetic field to the cellular culture plate (d1, d2).

## Description

### TECHNICAL FIELD

The present disclosure relates to the field of bioreactors for cell cultures. In particular, the disclosure also relates toa modular bioreactor for providing magnetic, magnetoelectric, stretching and electrical stimuli to cellular cultures and other biomedical applications through electronic controlled stimuli, based on permanent magnets and/or electromagnets.

### BACKGROUND

Currently, there is a large variety of bioreactors available for cellular culture, such as stirred tank bioreactors, rotational bioreactors, microfluidic devices and disposable systems with specific configurations such as wave bioreactors or Pneumatic Bioreactor Systems (PBS).

Bioreactors are usually used to provide an appropriate environment for cellular culture and/or to develop tissues from scaffolds (cellular supports) seeded with cells. It is possible to find some solutions (US 8,367,410 and US 2014/0038258) where chemical and electrical stimuli are applied in a way that they promote cellular proliferation and differentiation, as well as the regeneration of different tissues. The human body generates electric currents and fields in several places, which are recognised as relevant inputs for the treatment and regeneration of different tissues (such as bone, muscle and cartilage). In this way, some systems have been designed in order to satisfy this approach in the field of tissue engineering.

Similarly, the application of mechanical stimuli to promote proliferation and differentiation in animal cells and/or human cells has also been explored (US7,732,204 and EP 1 990 402) and, according to several presented solutions, it has been observed that cellular differentiation is also enhanced when cells are subjected to mechanical stimuli.

A large variety of bioreactors have been developed, in which some of them have the specific goal to provide cells with physical stimuli besides biochemical (WO 2013/185818 and US 2014/0038258). Regarding these documents, both are related to bioreactors that provide mechanical and electric stimuli to tissue engineering applications.

Different types of stimuli are relevant for tissue engineering, so many types of mechanisms have been developed to provide them. A modular solution has been proposed in the document US 20110136225A1, in order to provide mechanical and electrical stimulation as the previous one, as well as perfusion stimulation as well. It has been suggested the use of a polydimethylsiloxane layer adapted to hold the scaffold inside a cartridge, a perfusion pump module, a mechanical transmission module for stretching and rotational forces, and a signal generation module for electrical waves.

It is desirable to provide a physical and biochemical environment in order to develop *in-vitro* models, which can reproduce the human body conditions where cells and tissues are developed (*in-vivo*)*.* However, the systems and methods available currently do not provide the desired environment, required to mimic the *in-vivo* conditions.

The development of new materials, such as magnetostrictive and/or magnetoelectric materials, allow the transduction of a magnetic signal into a mechanical or electrical variation, respectively, allowing, in this way, the mechanical and electrical stimuli of cells through the magnetic field, when the scaffolds fabrication is based in these materials. At the same time, the construction of polymeric fibre scaffolds embedded with conductive fillers allow an appropriate environment for mechanical stretching and electrical conduction of current, allowing those stimuli to reach the cellular sites seeded in the material. Their large potential can only be explored with the availably of appropriate bioreactors.

Currently, as previously mentioned, there are some systems to provide mechanical and electric stimuli, but with very reduced offer when the stimuli is based on the use of magnetic field.

Regarding bioreactors that use magnetic fields, only a bioreactor has been reported which uses a stimulation method based on a diamagnetic force field in one or more regions of the culture space (US 7,482,154). Besides that one, regarding the patent application US 2014/0220672, it is possible to observe the development of an hardware for magnetic 3D culture, through the use of magnetic particles in order to move cells while not providing any other stimuli to the cells (mechanical or electrical).

Regarding US 7,553,662 document, it describes subjecting the cells to mechanical stretching. The mechanical stimuli are produced magnetically. It is reported the possibility to use magnetic particles connected to the growing tissue and subjected to a variable magnetic field, in which the particles would move with the field and may produce the referred mechanical movements.

Regarding US 2011/0136225A1 document, it describes a system with a central control, which communicates with individual bioreactors, each with a cartridge, a perfusion chamber, a pump and a gas exchanger, for sets for perfusion and electrical stimulation and other for perfusion and mechanical stimulation.

From all presented solutions, none describes a modular magnetic bioreactor which provide a cooperative magnetic, mechanical, electrical and magnetoelectric stimuli to the cellular support and to the cells themselves through permanent magnets or/and electromagnets.

These facts are disclosed in order to illustrate the technical problem addressed by the present disclosure.

### GENERAL DESCRIPTION

The bioreactors of the present disclosure allowed the development of new study models and cellular culture strategies, significantly improving their viability and functionality, offering a greater grade of consistency, reproducibility, predictability and robustness.

The present disclosure refers to a bioreactor system, comprising modular components in order to provide variable magnetic field and/or stretching force to supply stimuli to the cellular supports and to the cells, electrical impulses to cellular supports, simultaneously to the biochemical medium in which the cells are subjected in the culture plate. More specifically, it is described a magnetically driven bioreactor system, which uses magnetic field and magnetoelectric effect for driving mechanical and electrical stimuli to cellular culture and biomedical applications, promoting the cellular growth and differentiation through the electronically controlled stimulation, based on permanent magnets and/or electromagnets. These stimuli may be converted in mechanical or electromechanical signals with appropriate materials, such as magnetostrictive or magnetoelectric materials, respectively, as well as conductive materials as cellular supports (scaffolds). Similarly, these bioreactors may be used in other biomedical fields, such as controlled drug delivery and magneto-transfection, based on the previously mentioned principles. Particularly, this system is essential for the culture of cells subjected to electrical and mechanical solicitations during their activity, such as myoblasts or osteoblasts, among others.

The present disclosure relates to a modular magnetically driven bioreactor for applying stimuli to cell culture scaffolds comprising:
at least one cellular culture plate (d1, d2);
a first module comprising a support enclosure (a) and a magnetically inert cover (c) arranged above the support enclosure (a) for supporting the cellular culture plate (d1, d2);
an actuator module (b1, b2) arranged below the magnetically inert cover (c);
wherein said first module is arranged for receiving said actuator module (b1, b2);
wherein the actuator (b1, b2) comprises:
   a fixed platform, also referred as a fixed table,
   a movable platform, also referred as a movable table;
wherein said movable platform, also referred as a movable table, comprises a plurality of magnets for applying a movable magnetic field to the cellular culture plate (d1, d2).

in an embodiment, the modular magnetically driven bioreactor comprising:
at least one cellular culture plate,
a first module as a support enclosure and a magnetically inert cover arranged above the support enclosure for receiving the cellular culture plate,
a second or a third module;
wherein said first module is arranged for receiving a second or a third module;
wherein said second module comprises a magnetic field actuator arranged for generating a magnetic field, wherein said magnetic field actuator is placed below the magnetically inert cover and comprises at least two platforms, a first platform being fixed to the first module and a second platform arranged to be moveable by a motor and wherein said second platform is screwed to an equally spaced matrix table of permanent magnets; or
wherein said third module comprises a stretching actuator arranged for generating electro-stretching stimuli, comprising a lower mechanical apparatus comprising at least two platform rows of permanent magnets, said lower mechanical apparatus being arranged to change the distance between said rows; and holding clamps arranged for withstanding permanent magnets to follow the magnetic force provided and thus stretching a cellular scaffold being held on the culture plate.

In an embodiment, the first module is arranged for receiving the second module comprising the magnetic field actuator and the third module comprising the stretching actuator, , in particular the modular magnetically driven bioreactor comprising both the second module and the third module as interchangeable parts of a kit.

In an embodiment, the modular magnetically driven bioreactor system further comprises an electrical impulses module, preferably the electrical impulses module being arranged as an external box-like structure to be coupled to the back of the first module for applying electrical contact, said electrical impulses module comprises an electronically controlled circuit board and corresponding connectors to receive power from an electrical grid and provide communication from the bioreactor.

In an embodiment, the second module comprises a magnetic field actuator arranged for generating magnetic field, said magnetic field actuator is placed below the magnetically inert cover and comprises a motion mechanism in ball-screw assembly and at least two platforms, a first platform being fixed by screws and/or narrow fitting to the first module and a second platform arranged to be moveable alongside a screw axis actuated by a motor and said second platform comprises an equally spaced matrix of permanent magnets.

In an embodiment, the second and third modules further comprises electromagnets.

In an embodiment, the second module further comprises two sets of spindles actuated by a step motor and a ball bearings arranged to allow the second platform movement.

In an embodiment, the magnetic field comprises frequencies between 0.01 to 100 cycles/second.

In an embodiment, the third module further comprises a mechanical transmission from a motor to a ball screw through a nut by a timing belt with cogs for providing a linear pull of the scaffolds supports.

In an embodiment, the cellular culture plate comprises 24 to 48 wells and contains the scaffolds featuring magnetic properties, provided that the second module comprising the magnetic field actuator is the actuator.

In an embodiment, the cellular culture plate comprises scaffold support between two scaffold clamps arranged on opposite sides, two contact plates attached to the scaffold support, and wirings, provided that the third module comprising the stretching actuator is the actuator.

In an embodiment, the scaffold support comprises magnetostrictive and magnetoelectric elements, particularly magnetostrictive materials selected from cobalt ferrites, nickel ferrites and Terfenol-D, coated with piezoelectric or non-piezoelectric polymers or magnetoelectric materials or fibrous seeded with skeletal muscle cells.

In an embodiment, the piezoelectric polymer is selected from poly(vinylidene fluoride) or polylactic acid and the non-piezoelectric polymer is selected from Poly(vinylidene fluoride) in the alpha phase or poly(lactic-co-glycolic acid).

T In an embodiment, the piezoelectric polymer is embedded with conductive particles selected from graphene, carbon nanotubes or ionic liquids.

In an embodiment, the wirings of the cellular culture plate of the third module comprises a material selected from gold, stainless steel, platinum, titanium or palladium and the contact plates are selected from

In an embodiment, the platform of the second module and the platforms of the third module are made of a material selected from a list consisting of: stainless steel; aluminium; or polyethylene or nylon or a not- ferromagnetic plastic or not-ferromagnetic metal or ceramic; or combinations thereof.

In an embodiment, the support enclosure of the first module comprises a thin layer display of liquid crystal transistors and a capacitive keyboard that enables an interface with the device.

In an embodiment, it is disclosed a modular magnetically driven bioreactor, preferably a system that may comprise:
a module I: at least a main enclosure (a) to work in a modular way that communicate together provided with: a man-machine interface unit provided of openings to receive the internal modules (Module II and III one at each time) provided with:
   i) a support enclosure (1);
   ii) mechanical modules (b1) or (b2), see below, which work with different types of standard culture plates (4), (b1) with (d1) and (b2) with (d2);
   iii) a cover placed above the enclosure upper surface;
   iv) a side view with a trapezoid shape (or any other shape allowing user interface and magnetic interface with cell culture plates) with a thin layer display of liquid crystal transistors 2; and
   v) a capacitive keyboard that enables the user interface with the device, keeping the waterproof feature of the system;
a module II: magnetic field actuator (**b1**) consisting on a mechanical apparatus, which is placed immediately below the cellular culture plate (**d1**) with a motion mechanism in ball-screw assembly and is composed mainly by at least two platforms, one is fixed by screws and/or narrow fitting to the Module I, and the other moves alongside the screw axis actuated by a motor;
a module III: Stretching actuator (**b2**) also consisting on a mechanical apparatus, which is placed below the cellular culture and scaffold holding clamps, which are placed in the cellular culture plate (**d2**) interior, provided with at least two main components:(i) a lower mechanical apparatus which changes the distance between at least two rows of permanent magnets; and (ii) the holding clamps withstanding permanent magnets follow the magnetic force provided and thus stretching the cellular scaffold being held; and
a module IV: The electrical impulses module (**e**) consists of an external box-like structure coupled to the back of the main Module I and in which there is a holding of electronically controlled circuit board and corresponding connectors to receive power and communication from bioreactor.

In an embodiment, there may be at least two modular configurations of the methods and system:
a) magnetic stimuli (11) method provided by the set of: Module I (a), Module II (b1), cover c and culture plate (d1);
b) electro-stretching stimuli (11) method provided by the set of: Module I (a), Module III (b2), Module IV (e), cover (c) and respective culture plate (d2);
c) at least a magnetic table (5) (Module II b1) or (15) (Module III b2), where a group of permanent magnets and/or electromagnets are the active components; and combinations thereof.

In an embodiment, the type of stimuli intended may determine the mechanical module (b1 or b2) to be used in the system and are generated by physical means selected from the group consisting of: magnetic, magnetostrictive, magnetoelectric or conductive materials or any other similar material.

In an embodiment, the magnetostrictive element may be cobalt ferrites, nickel ferrites and Terfenol-D, among others, optionally coated by a non-piezoelectric material, in which case only the mechanical stimuli is transferred to the cells.

In an embodiment, the magnetic tables (5 and 15) material may be selected from of stainless steel or aluminium or polyethylene or Nylon or other plastic or metal that does not feature ferromagnetic properties and are controlled electronically by the control system (13).

In an embodiment, magnetic fields may be produced by permanent magnets (11) generating a continuous magnetic field or electromagnets generating an alternated magnetic field display a cylindrical parallelepiped, toroidal format or any other shape in function of the designed geometry of magnetic field lines.

In an embodiment, the scaffold materials may be obtained by preparing a solution with magnetoactive/conductive materials and polymer and thus, processed into film, fibres or 3D constructs through different methods such as additive manufacturing technologies, doctor blade, electrospun fibres and template removal, respectively.

In an embodiment, the magnetic table (5) moves along of at least one transversal axis by the use of a nut (8) and a screw (7) assembly attached to the magnetic table (5) and slides relying in an assembly of spindles (9) and ball bearings (10) to reduce attrition and the mechanical heat production.

In an embodiment, the spindles (**9**) may be actuated by a step motor (6), a servomotor, or other similar motor, which grants a precise positioning control, as well as fast enough speeds which does not produce a thermic effect.

In an embodiment, the stretching mechanism (Module III b2) stimuli may be obtained using two lines of permanent magnets, electromagnets or a combination of them (14) placed in between a static and a movable magnetic table (15), which will move the magnetic force in a controlled distance at a desired frequency, being controlled electronically by the control system (13), pulling the scaffolds (18) attached to two clamps (20).

In an embodiment, the most suitable materials for electro-stretching scaffolds may be based on polymer materials, such as poly (vinylidene fluoride) or poly (lactic acid), among others, embedded with conductive particles such as graphene, carbon nanotubes or ionic liquids, among others which allow the transduction of the available stimuli to beneficial stimuli for the cellular proliferation and differentiation, such as different biochemical effects through the magnetic stimuli by the utilization of materials with appropriate magneto-chemical and/or magneto-rheological response, among others.

In an embodiment, both systems may fit in the same main bioreactor enclosure (a), which may require a different mechanical transmission from motor (6) to the ball screw (7) through the nut (8), by using a timing belt with cogs (16), providing the linear pull of the scaffolds supports.

In an embodiment, the option by the system to be used is performed by the user, by replacing the magnetic table (5), removing the upper cover of the waterproof enclosure (12), making the magnetic table (5) accessible for replacement. The user may replace the cover in order to use with non-standard culture plates.

In an embodiment, the electrical impulse contact plates (19) attached by screw to the scaffold supports are provided through biocompatible conductive materials such as gold, stainless steel, platinum, titanium or palladium, among others in the wiring (17).

In an embodiment, the scaffold supports can be worked in biocompatible materials such as Teflon and acrylic, among others, as well as being 3D printed in a biocompatible plastic such as polylactic acid.

In an embodiment, the external electrical module (e) is built with waterproof materials such as stainless steel, acrylic, nylon with proper isolation rubbers in the each of the joints, comprising at least a waterproof enclosure (24), waterproof power and communications connectors (22), a control system (23), which communicates with the main control system (13) and controls a group of parameters that define the type and format of the generated stimuli.

In an embodiment, the electrical connections may be performed by the use of stainless steel wires or other stainless biocompatible material.

In an embodiment, the materials used for the construction of the different mechanical parts are preferably stainless steel, aluminium, polyethylene, or other plastic or metal, which does not display any ferromagnetic properties, which are not susceptible to corrosion in the culture environment and satisfy the functional requirements of the system.

In an embodiment, it is possible to define a pattern of chained cycles, the time in which are produced the stimuli as well as the time with no stimuli, as well the stimuli waves shape to be square, triangular, sinusoidal or any other wave type which reveals to be necessary for the cellular stimuli and the frequency, amplitude and offset allowing the stimuli reproduction to be the closest possible way as natural as possible.

In an embodiment, the electronic control Module is provided by the electrical grid (A), where through an interface power system (B) to convert the voltage levels in a appropriate values and an electronic control system (C) which performs the mechanical displacement system actuation (E), as well as the magnetic or electromagnetic actuation of said system actuation (E) and any extra module (H).

In an embodiment, wherein alternatively, the amplitude and the shape of the generated magnetic field produced by the electromagnets, in the case of being used, varying the electrical current that makes the electromagnets actuate, through a power electronic control system (D).

In an embodiment, the shape of the generated field may be measured by a group of Hall Effect, force and displacement (F) sensors, installed preferably in the magnetic table (5).

### BRIEF DESCRIPTION OF THE DRAWINGS

The following figures provide preferred embodiments for illustrating the disclosure and should not be seen as limiting the scope of invention.
**Figure 1****:** shows the modular bioreactor in its entirely, as well as all available modules that can be used accordingly to intended type of stimuli and to which type of culture plates.
**Figure 2****:** illustrates a schematic exterior perspective with the main control module of the bioreactor system.
**Figure 3****:** displays a schematic of a cut perspective, showed by the cut line **I-I** of **Figure. 2**, where the diverse components (external and internal) are visible, as well as the interconnections that compose the magnetic bioreactor with its variable magnetic field module.
**Figure 4****:** displays a schematic perspective of the variable magnetic module mechanical displacement.
**Figure 5****:** displays a schematic of a cut perspective showed by the line **I-I** of **FIG. 2**, where the diverse components (external and internal) are visible, as well as the interconnections that compose the magnetic bioreactor with its magnetic force pulling interior module and electric impulses external module.
**Figure 6****:** displays a schematic perspective of the stretching module mechanical displacement.
**Figure 7****:** displays a standard culture plate for stretching with respective scaffold supports and electrical contacts.
**Figure 8****:** displays a schematic cut perspective of the electrical stimuli module waterproof enclosure.
**Figure 9****:** shows the schematic description of the system's control configuration, where the several control variables are schematized, as well as the several combinations possible between them.
**Figure 10****:** shows the schematic description of the electronic control system, which conducts the system mechanical displacement actuation as well as the magnetic and magnetoelectric triggering.

### DETAILED DESCRIPTION

The present disclosure refers to a bioreactor system that provides magnetic, magnetoelectric and electro stretching stimuli to the cellular supports and to the cells, simultaneously to the biochemical medium in which the cells are subjected in the culture plate.

More specifically, the bioreactor system of the present invention comprises modular components in order to provide variable magnetic field, stretching force and electrical impulses to cellular supports, in which the cells are cultivated. For a preferable embodiment of the present invention, said cellular support is preferable a compound provided by magnetostrictive particles selected from the group of fibrous, film or 3D shapes of polymers hydrogels, or ceramic materials. However, these examples are not limitative of the present invention. A person skilled in the art knows that any other compound usually used for this purpose can be also used in the present invention.

The stimulus provided by the variable magnetic field internal module, triggers, in the support, which contains magnetostrictive particles, a mechanical variation due to the magnetostrictive effect. If the support where the particles were introduced is also piezoelectric, the particles mechanical variation will induce an electrical response in the material (induced variable voltage) that will affect the growing cells seeded in these supports.

By using the stretching module together with the electrical module, the system is able to provide direct current impulses on the scaffold materials (fibrous supports) and cells as well as stretching stimuli using scaffold supports (holding the scaffolds), which are pulled by magnetic force.

As result of the bioreactor herein developed, the cells may be grown in a system, which mimics the existing stimuli in the human body, and are essential to an appropriate tissue engineering of cells such as mesenchymal, skeletal, neuronal and muscular, among others.

This new system allows the simultaneous application of different stimuli to the cells or individual stimuli through the combination of scaffolds composed by magnetostrictive particles embedded in piezoelectric such as poly(vinylidene fluoride) and polylactic acid, among others or non-piezoelectric polymers such as Poly(vinylidene fluoride) in the alpha phase, or poly(lactic-co-glycolic acid), among others. It is essential for an appropriate knowledge of the cells, as well as for the differentiation and functional growth of specific types of cells, including bone, muscle or cartilage cells among others that need one or other stimuli, or a combination of them.

The culture environment provided by the present system is essential for the culture of cells subjected to electrical and mechanical solicitations during their activity, such as myoblasts or osteoblasts, among others.

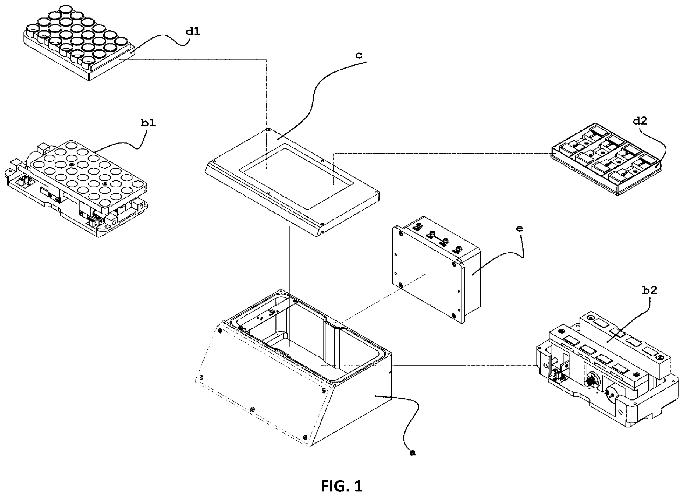

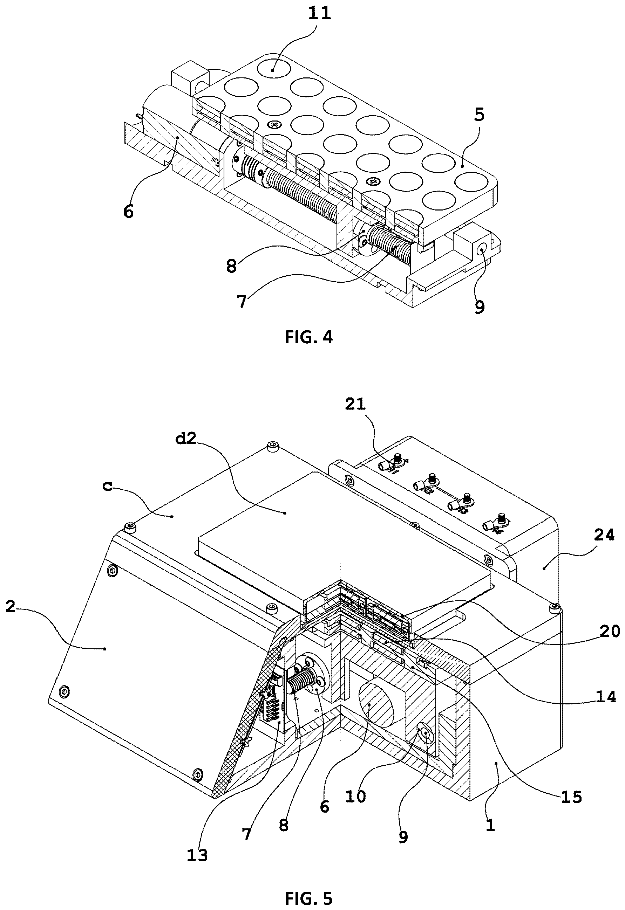

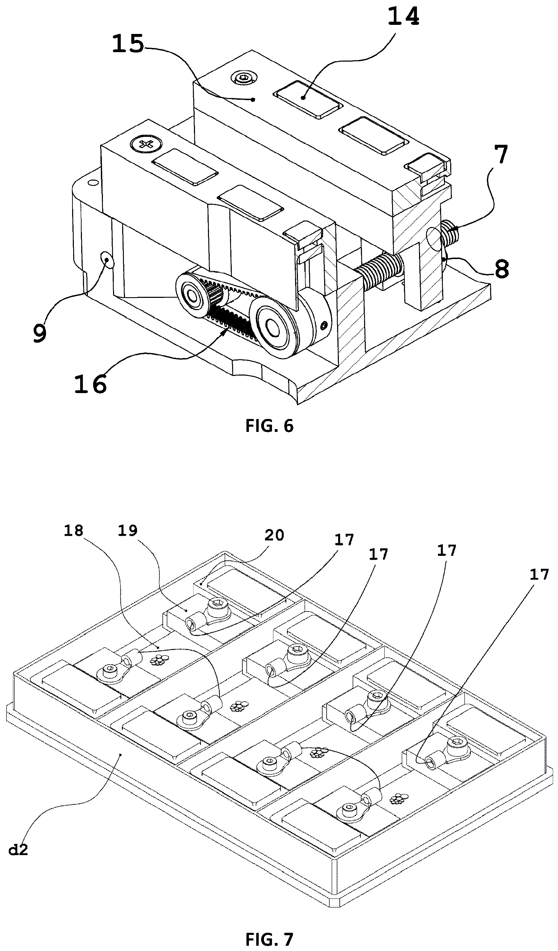

In general lines, as shown in Figure 1, the bioreactor of the present invention comprises two types of mechanical configurations according to type of scaffolds used and type of stimuli intended: at least a main enclosure (a) which works with interchangeable components:
- Module I - it is the main Module and comprises a box-like structure provided of openings to receive the internal modules (Module II and III one at each time), depending on stimuli type to be used. The internal modules are attached to the main module by a moving mounting devices, like as screws. This module presents a side view with a trapezoid shape, or any other shape allowing user interface and magnetic interface with cell culture plates, where within the single face with inclination lies the user interface (Display (transistor) and Waterproof Keyboard), however it can take any other format. At the top of the system lies a magnetically inert cover (**c**), under which the cellular culture is kept above the mechanical modules II and III;
- Module II - Magnetic field actuator (**b1**) consisting on a mechanical apparatus (**FIG. 4**), which is placed immediately below the cellular culture plate (**d1**). It presents a motion mechanism in ball-screw assembly and is composed mainly by at least two platforms, one is fixed by screws and/or narrow fitting to the Module I and the other moves alongside the screw axis actuated by a motor. At the top of the moving platform an equally spaced matrix of permanent magnets are magnetically fixed by top and bottom permanent magnets; that is, at the top of the moving platform, the magnetic table is an equally spaced matrix of permanent magnets, being fixed magnetically in each point of the matrix by a gap between a magnet in the top and another in the bottom of the table, holding each other against it;
- Module III - Stretching actuator (**b2**) also consisting on a mechanical apparatus (**FIG. 6**), which is placed below the cellular culture and scaffold holding clamps, which are placed in the cellular culture plate (**d2**) interior. Thus, the module III presents at least two main components:(i) a lower mechanical apparatus which changes the distance between at least two rows of rectangular permanent magnets or any kind of element which are more suitable for an aligned straight motion than disk shaped magnets; and (ii) the holding clamps withstanding permanent magnets follow the magnetic force provided and thus stretching the cellular scaffold being held; and
- Module IV - The electrical impulses module (**e**) consists of an external box-like structure coupled to the back of the main Module I and in which there is a holding of electronically controlled circuit board and corresponding connectors to receive power and communication from bioreactor. Simultaneously it provides electrical contacts to wire into the scaffold holding clamps own electrical contacts present in the cellular culture plate (**d2**). This module IV enables the application of controlled continuous DC current or square, triangular, sinusoidal current waves at a user-defined frequency, throughout the scaffolds seeded with cells.

(ii) at least a totally waterproof enclosure(s) built with waterproof materials such as stainless steel, acrylic, nylon with proper isolation rubbers in the each of the joints or any other material provided with this characteristic, that assures the proper functioning of the bioreactor throughout the time, as the system will be operating inside a greenhouse with high humidity rate; and
(i) at least a main control electronic system, which communicates with others sub-control systems.

Further, the bioreactor allows:
a) To be assembled according to the intended stimulus, by replacing the interior mechanics in the Module I interior: for magnetic variation on cellular culture plate (**d1**) by using Module II (**b1**) - (**a+b1+c+d1**); or for magnetic pulling on culture plate (**d2**) the stretching actuator **(b2)** - (**a+b2+c+d2**). Further, an extra electrical impulse module (**e**) for controlled current impulses on scaffold clamp contacts (**17**) can be added to the last configuration, resulting in **a+b2+c+d2+e** assembly.
b) Production of a combination of continuous magnetic fields -to evaluate its effect during the cellular growth or to stimulate the controlled release of active elements. Alternatively, variable magnetic fields evaluate the previous mentioned parameters in dynamic conditions or stimulate the mechanical variations of the magnetostrictive and/or magnetoelectric particles of scaffolds. Both methods will induce magnetic variations and/or electric during cellular culture.
c) Define through the electronic system and the construction parameters, frequency, amplitude and stimuli offset, according to intended conditions to the essay, in a way that essays can be carried out varying systematically the previously mentioned parameters and evaluate their effect during the cultures.
d) Define through the electronic system and the construction parameters, the applied stimuli form, which can be based in square, triangular, sinusoidal and any other wave that reveals to be necessary during the essay. These types of wave-forms allow obtaining cellular cultures in different dynamic stimuli conditions in order evaluate the cellular response.
e) Define a group of chained cycles of stimuli and/or repose of the cells accordingly to the intended conditions for the essay.
f) Produce, within the configuration of stimuli parameters previously mentioned, a group of magnetic fields and/or magnetoelectric capable of stimulating the scaffold used in the essay.
g) Use of scaffolds for cellular cultures based in materials and/or particles, which respond to magnetic and/or magnetoelectric stimuli, including magnetostrictive particles coated with piezoelectric or non-piezoelectric polymer, and/or introduced in a polymer with different geometries (spheres, fibres, membranes or 3D scaffolds), so different parameters can be evaluated in their influence on the cellular growth.
h) Move magnetic particles, in a single direction or alternated movement, according to the essay objective, as well as controlled drug delivery or magneto-transfection.
i) Study the drug delivery and/or growth factors, proliferation and cellular differentiation, among other relevant applications in which the stimuli allow to be applied.

These stimuli are achieved through the magnetic modular bioreactor that provides magnetic stimuli in conventional culture plates, where the culture scaffolds are placed, enabling the cellular differentiation/proliferation with magnetoactive cells or scaffolds. In short, the alternated magnetic field produced with permanent magnets displacement or electromagnetic actuators (fixed electromagnets with alternated current) enable magnetostrictive scaffolds to vibrate at the field frequency, usually sub-10 Hz frequency resembling the usual slow motion of the human body. Furthermore, the scaffolds can be composed by different materials, and, in case a piezoelectric material is coupled to the magnetostrictive one, the magnetostrictive vibration will stimulate the piezoelectric material generating a varying surface charge, through the magnetoelectric effect.

For scaffolds being fabricated with magnetostrictive and/or magnetoelectric materials, the magnetic stimuli are transduced into mechanic and/or electric signs. For fibrous scaffolds seeded with skeletal muscle cells, the electro-stretching stimuli, is generated by the magnetic pulling of Module III (**b2**), and the external current controller present in module IV (**e**). As previously mentioned, the stimulation of the bioreactor is controlled electronically, based in a group of permanent magnets and/or electromagnets in variable number, depending on the number of wells of the used culture plate (**d1** or **d2**).

In a preferable embodiment of the present invention, Module II (**b1**) uses the permanent magnets approach for 24 or 48-wells culture plate, harbouring one magnetic table which can be replaced for each number and well sizes of the desired culture plate (24 or 48) in this case), as they must comply with the diameters of the culture plate wells. Alternatively, in second preferable embodiment of the present invention, in the case of stretching stimuli, by using the mechanical Module III (**b2**), it requires a use of a 4-wells rectangular culture plate, as the standard, in order to fit the clamps and provide stretching space inside the cell medium.

In the Module II (**b1**) is also possible to generate other controlled stimuli from the magnetic stimuli, such as mechanical stimuli, which act in diverse cellular processes, since the proliferation to the transcription and organogenesis due to gene expression changes, signalling of the second messenger (per example, intracellular Ca²⁺), focal adhesions and internal remodelling of the cytoarchitecture.

In addition, the electromechanical stimulation affects the cellular adhesion, orientation and migration, and influences the regulation of morphologic and phenotypic processes involved in the cell proliferation and differentiation, resulting in a modification of cellular membrane voltage at superficial and internal level, the synthesis of glycosaminoglycans, the expression of extracellular genes, and the reduction of inflammatory mediators, among others.

Finally, other applications such as the movement of magnetic nano- and microparticles, with or without functionalization, may be essayed, with applications since the magneto-transfection and/or drug delivery, for example.

In view of the features above, the bioreactor enables a new platform to create innovative strategies for tissue engineering, controlled drug delivery, microbial cultures, which support magnetic, electrical and mechanical stimuli, among others.

The introduced innovation and enhancement by this technology is based on the fact of its implementation lead to obtaining more realist experimental results; this means, closer to the *in vivo* results, as also the planning and execution of new experiences towards a greater and better knowledge of biological and biomedical systems. Based on this, the system of the present invention will reduce the dependence on the use of animal models, which, with the reduction of the animal sacrifices, and the respective ethical dilemmas involved, will lead to a decrease of the expenses involved in the use of these models, as well as the increase and greater possibility of tests when transferred to a real application *in vivo.* Finally, the developments and advances performed can be transferred to innovative therapies *in vivo*, given the feasibility of applying magnetic fields inside the body.

Therefore, in a preferable embodiment of the present invention, a modular bioreactor system is described as a device that grants a group of magnetic, magnetoelectric, mechanical and electrical stimuli to cellular supports (with fibrous, film or 3D shapes) and to the seeded cells, based in groups of permanent magnets and/or electromagnets controlled by an electronic system.

The magnetic bioreactor system comprises:
a. a main enclosure (Module I **a**) to work in a modular way that communicate together, according to user needs,
   1. a man-machine interface unit comprising a thin layer display of liquid crystal transistors **2**, named TFT (Thin Film Transistor) based display or LCD (Liquid Crystals Display) placed in Module I, for information display to the user of the experiment parameters as well as their state; and
   2. a capacitive keyboard or any other keyboard with other technology that enables the user interface with the device, keeping the waterproof feature of the system.
b. at least two modular configuration methods: (i) magnetic stimuli method provided by the set of: Module I (**a**), Module II (**b1**), cover **c** and culture plate (**d1**); and (ii) electro-stretching stimuli method provided by the set of: Module I (**a**), Module III (**b2**), Module IV (**e**), cover (**c**) and respective culture plate (**d2**);
c. at least a magnetic table **5** (Module II **b1**) or **15** (Module III **b2**), where a group of permanent magnets and/or electromagnets are placed, which produce continuous and/or alternated magnetic fields;
d. at least a displacement mechanical system based on ball screw actuated by a step motor, producing at least a variable magnetic field in space.

The magnetic table (5) and (15) being of stainless steel or aluminium or polyethylene or Nylon or other plastic or metal that does not feature ferromagnetic properties.

By the use of the alternated magnetic field generated by the Module II, this system provides the magnetic stimuli to the used support (d1), and in consequence, through the used materials as cellular supports. The magnetic stimuli are generated by physical means selected from the group consisting of: magnetic, magnetostrictive, magnetoelectric or conductive materials or any other similar material for this purpose will provide the aforementioned stimuli to the cells seeded in those supports.

Those scaffold materials are obtained by preparing a solution with magnetoactive/conductive materials and polymer and thus, processed into film, fibres or 3D constructs through different methods such as additive manufacturing technologies, doctor blade, electrospun fibres and template removal, respectively. Depending on the type of tissue structure to mimic, the scaffold processing method will take a preponderant role, as examples, for a bone porous structure culture, a 3D construct through template removal is suitable, whereas for muscle tissue culture, electrospun fibres are the ones that best mimic such tissues.

This way, the most suitable supports (scaffolds) will be preferably based in magnetostrictive and magnetoelectric composites, which consist in the combination of magnetostrictive materials (such as cobalt ferrites, nickel ferrites and Terfenol-D, among others) and fibrous material, like as a piezoelectric (Poly (vinylidene fluoride) or polylactic acid, among others) and other similar materials. Any other polymer, hydrogel or ceramic can be also used as support scaffold material. However, a person skilled in the art knows that the magnetostrictive materials, as well as the polymers described herein are merely examples and they are not limitative options of the scope of invention.

When composite magnetoelectric supports (composed by magnetostrictive and piezoelectric materials), are submitted to a magnetic stimuli provided by the alternated magnetic field of the bioreactor, a mechanical deformation is induced by the magnetostrictive component, which results in an electrical polarization, due the piezoelectric effect of the other material component.

Thus, the magnetic actuation capability of these types of composites allows magnetic, mechanical and electrical stimuli to the cells simultaneously or individually based on the appropriate selection of materials for the cellular support.

Given the magnetostrictive element (such as cobalt ferrites, nickel ferrites and Terfenol-D, among others) coated by a non-piezoelectric material, only the mechanical stimuli will be transferred to the cells.

By the use of the stretchable module (Module III (**b2**)) and external electric module (Module IV (**e**)), the bioreactor provides stretching and electrical stimuli to the used scaffold in the culture plate **(d2).** Each scaffolds lie attached to two clamps (**20**) inside the cellular culture medium (containing oxygen and nutrients) holding it for stretching while providing the electrical connections directly to the scaffolds and cells.

The most suitable materials for electro-stretching scaffolds will be based on polymer materials, such as poly (vinylidene fluoride) or poly (lactic acid), among others, embedded with conductive particles such as graphene, carbon nanotubes or ionic liquids, among others. However, other materials may be used in the supports, which allow the transduction of the available stimuli to beneficial stimuli for the cellular proliferation and differentiation, such as different biochemical effects through the magnetic stimuli by the utilization of materials with appropriate magneto-chemical and/or magneto-rheological response, among others.

However, a person skilled in the art knows that the fibrous material is optional to have conductive particles when for mechanical stretching only.

**FIG. 1**, which corresponds to the modular schematic of the system in its entirely, being composed by trapezoidal main enclosure (**a**) where mechanical modules (**b1**) and (**b2**) are placed inside accordingly to the type of stimuli intended. Each mechanical module, (**b1**) and (**b2**), works with different types of standard culture plates, (**b1**) with (**d1**) and (**b2**) with (**d2**). These culture plates are placed above the enclosure upper cover (**c**) as showed in **Fig. 5****.** The (**b2-d2**) configuration is designed to work together with the electrical impulses external module (**e**).

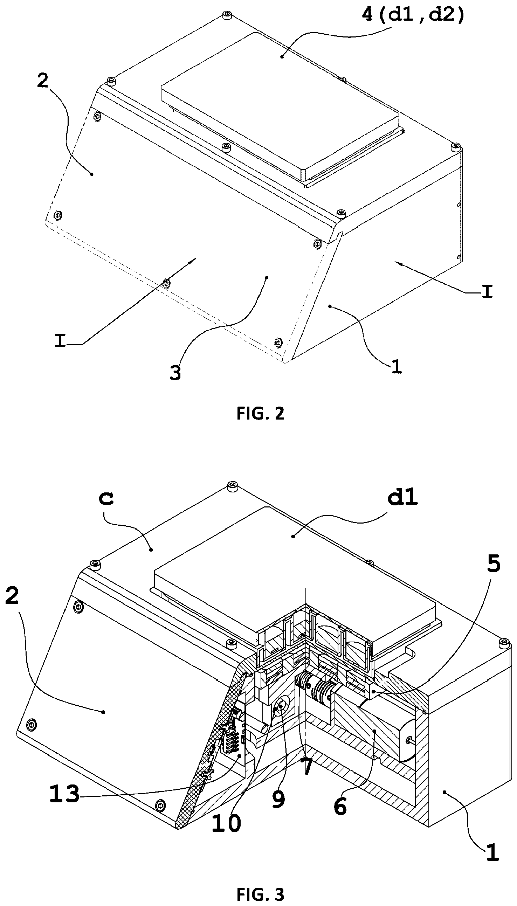

**FIG. 2**, which corresponds to the schematic perspective of the bioreactor system, being composed by a support enclosure (**1**) placed inside of the trapezoidal main enclosure a. Referred support enclosure 1 serves for housing the diverse internal components of the system, characterized for being a total waterproof enclosure. The characteristics and state of the stimuli group at which the cellular support is subjected is visible through a thin layer display of liquid crystal transistors (**2**), where the user interface is done by a capacitive keyboard (**3**), both placed behind a transparent material such as glass or acrylic plate, allowing the system to be totally waterproof. The culture medium, the cellular support and the cells, are placed in the culture plate (**4**), which can be either (**d1**) or (**d2**) as showed in **FIG. 1**, corresponding to a conventional commercial culture plates (without any special features for this bioreactor) repeatedly used for this end.

A person skilled in the art knows that the format and number of wells in the culture plate, such as (**d1**), may be many, suiting the needs of the users of the magnetic bioreactor system. On the other hand, the culture plate (**d2**) will have a fixed number of four large rectangular wells in order to fit the scaffold clamps (**20**) while providing the stretching margin required.

Through the **FIG. 3**, which relates to a schematic cut perspective indicated by the cut line **I-I** of **FIG. 2**, the system equipped with the alternated magnetic field module (**b1**), several internal components are visible, as well as their connections which compose the alternated magnetic field of the bioreactor system assembly (**a+b1+c+d1**).

As the objective is to enable a group of magnetic, mechanical and/or magnetoelectric stimuli to the cellular support and to seeded cells inserted in the culture plate (**d1**), this effect is achieved by the control of intensity, direction and position of magnetic field generated by the magnetic table (**5**). These fields behaviour is explained below.

The necessary fields can be obtained using the permanent magnets and/or electromagnet groups (**11**) (**Fig. 4**) positioning according to the culture wells pattern in the culture plate (**d1**) used and the magnetic field value in function of the desired magnetic field, or making use of the positioning of electromagnet groups (**11**). The magnetic table (**5**) is controlled electronically by the control system (**13**), although it may display a different pattern from the one illustrated in the figure (**Fig. 3**), depending on the number of cylinder wells, following a culture plate type such as (**d1**). The magnetic fields also may be produced through other materials or similar systems, which allow the generation of continuous and alternated magnetic field with the desired amplitude.

The permanent magnets generate a continuous magnetic field and electromagnets (**11**) which generate an alternated field display a cylindrical format, although they can display other formats like parallelepiped, toroidal, or any other shape that suits the purpose, in function of the designed geometry of magnetic field lines to a given functioning of the bioreactor.

The permanent magnets or electromagnets (**11**) display a size according to the culture plate wells, but there is no obligation to obey this relation, there is the possibility to use other relations or designs in function of magnetic field (amplitude and magnetic field line geometries) intended to be applied.

**FIG. 4** corresponds to the schematic of the mechanical displacement system as well as the magnetic system (Module II-**b1**). The mechanism enables the system the feature to move the magnetic table (**5**) along the transversal axis, which allows the generation of an alternated magnetic field in the space along the axis. If intended, the magnetic field can be generated following two or three axis for other innovative effects in given cultures and/or biomedical applications.

This movement is obtained using a nut (**8**) and a screw (**7**) assembly, through the rotation of screw system, where the nut (**8**) is mechanically attached to the magnetic table (**5**), enabling the displacement of the magnetic table (**5**). Once it is necessary a level of precision in the mechanical displacement, a decision to use a group nut (**8**) and screw (**7**) in ball screw, which allows high precision and low wear with cyclic movements, it is important to underline that a person skilled in the art could employ other similar mechanism that enables the same movement.

The sets of spindles (**9**) are actuated by a step motor (**6**), a servomotor, or other similar motor, which grants a precise positioning control, as well as fast enough speeds, which enable the system to move the magnetic table (**5**) at high enough frequencies, thereby granting the possibility to generate alternated magnetic fields comprehending frequencies between 0.01 to 100 cycles/second, depending of the cellular culture type.

It is also worth highlighting that the motor actuation as well as the positioning mechanical system should not produce a thermic effect that compromises the cell viability for intended applications, such as in the cellular culture cases where the thermal restriction are very stringent (never go over 36-37ºC), as it will be described below.

In order to avoid excessive mechanical struggle as well as quick wear of the mechanical components, the magnetic table (**5**) slides relying in an assembly of two sets of spindle (**9**) and ball bearings (**10**), which allows a movement with reduce attrition, reducing mechanical heat production.

**FIG. 5** relates to a schematic cut perspective indicated by the cut line **I-I** of **FIG. 2**, the system equipped with the stretching mechanical module III-**b2** several internal components are visible, as well as their connections which compose the electro-stretching bioreactor system.

As the objective is to enable electro-stretching stimuli through electrical connections and magnetic pulling to the cellular scaffolds and to seeded cells inserted in the culture plate (**d2**), this effect is achieved by the control of electric waveform and frequency, and mechanical intensity, direction and displacement distance of the stretching system, which applies a force in the scaffolds as, is explained below.

The stretching mechanism (Module III b2) stimuli is obtained using the necessary displacement (for example, up to 15% of the scaffold size) using at least two lines of permanent magnets, electromagnets or a combination of them (**14**) placed between a static and a movable magnets support, magnetic table (**15**), which will move the magnetic force in a controlled distance at a desired frequency controlled electronically by the control system (**13**), pulling the scaffold clamps (**20**). The electrical current stimuli can be placed in an external module IV (**e**), which is composed by a waterproof smaller case (**24**), having the electrical contacts built with biocompatible metal materials, thus the use of stainless steel screws not showed in the Figure 5 and nuts (**21**) are required to provide conductivity and biocompatibility. However, other biocompatible electrical transmission material can be used, such as metals such as palladium, gold or platinum offer the required conductivity and the biocompatible feature as well.

The permanent magnets or electromagnets or a combination of them (14) display a size according to the culture plate (d2), but there are no obligation to obey this relation, there is the possibility to use other relations or designs in function of magnetic pulling force area permitted by the culture plate (d2).

**FIG. 6** corresponds to the Module III-**b2** similar mechanical system to Module II-**b1** of **FIG. 4**, sharing similar components, but working in the longitudinal axis in order for both systems fit in the same main bioreactor enclosure (**a**), which requires a different mechanical transmission from motor (**6**) to the ball screw (**7**) through the nut (**8**), by using a timing belt with cogs (**16**). Thus, providing the linear pull of the scaffolds supports.

The user can opt by making use of one or another solution (permanent magnets or electromagnets), or can opt by a use of a mixed system, taking advantage of the different features of both systems, such as the continuous magnetic field of permanent magnets or the alternated magnetic field of electromagnets. A preferable embodiment of the present invention uses the permanent magnets displacement due to the lower electrical current requirements, and thus less heat production in the system interior.

This change is performed by the user, by replacing the magnetic table (**5**), being necessary to remove the upper cover of the waterproof enclosure (**12**) (removable and waterproof), making the magnetic table (**5**) accessible for replacement, or also replace the cover in order to use with non-standard culture plates.

**FIG. 7** displays the main configuration for electro stretching stimuli, inside the culture plate (**d2**), which is achieved by placing the scaffold material (**18**) between two scaffold clamps (**20**) embedded with permanent magnets or other ferromagnetic material. The scaffold clamps (**16**) can be pulled by an exterior magnetic force provided by permanent magnets or electromagnets or a combination of them (**14**) in the interior of main bioreactor enclosure (**a**), transmitting a remote pulling force, which does not contaminate the cellular environment.

The electrical impulses are provided through biocompatible conductive materials such as gold, stainless steel, platinum, titanium or palladium, among others in the wiring (**17**), among others as biocompatible electrical transmission when invasive on cellular culture. The scaffold clamps (**20**) can be worked in biocompatible materials such as Teflon and acrylic, among others, as well as being 3D printed in a biocompatible plastic such as polylactic acid.

The contact plates (**19**) attached by screw to the scaffold supports, just like the wiring must be conductive and biocompatible comprehending the same possible materials such as gold, stainless steel, platinum, titanium or palladium, among others as biocompatible electrical transmission when invasive on cellular culture.

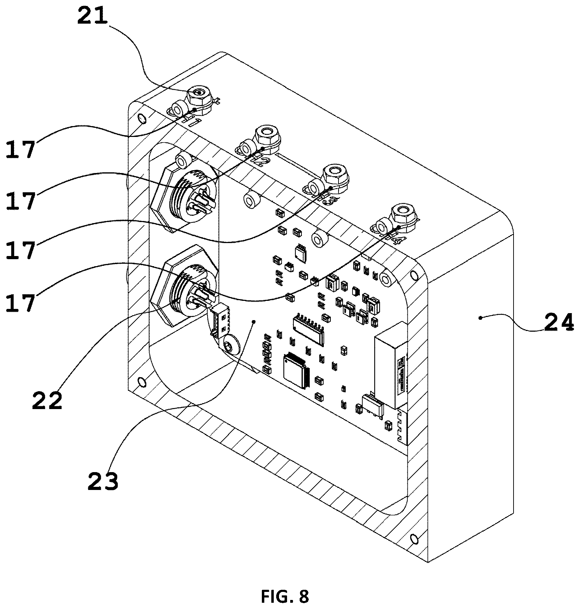

**FIG. 8** illustrates the interior components of the external electrical module (**e**), composed by a waterproof enclosure (**24**), waterproof power and communications connectors (**22**), a control system (**23**), which communicates with the main control system (**13**) and controls the current stimuli being applied to the cells seeded in the scaffolds (**18**).

The electrical connections are performed by the use of stainless steel wires or other stainless biocompatible material from connections (**21**) to (**17**). The system may apply the electric current stimuli in series or parallel according to control system and electrical connection configurations.

The materials used for the construction of the different mechanical parts aforementioned are preferably stainless steel, aluminium, polyethylene, or other plastic or metal, which does not display any ferromagnetic properties, which are not susceptible to corrosion in the culture environment and satisfy the functional requirements of the system.

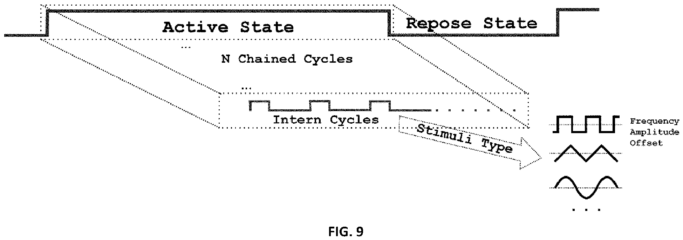

In the described system, it is possible to control through the electronic control system and respective configuration, a group of parameters that define the type and format of the generated stimuli, in **FIG. 9** the schematic description of the system's controlled configuration is displayed.

Thus, the system offers the possibility to define a pattern of chained cycles where it is possible to define the time in which are produced the stimuli as well as the time with no stimuli, allowing the cells present in the culture to get the stimulus in a way closer to their real environment.

On the other hand, it is possible to define as well the stimuli waves shape to be square, triangular, sinusoidal or any other wave type which reveals to be necessary for the cellular stimuli. For each wave shape selected is also yet possible define the frequency, amplitude and offset, intending with these parameters allowing the stimuli reproduction to be the closest possible way as natural as possible, as well as allowing scientific experiments with a systematic variation of the most relevant parameters involved in each process under investigation.

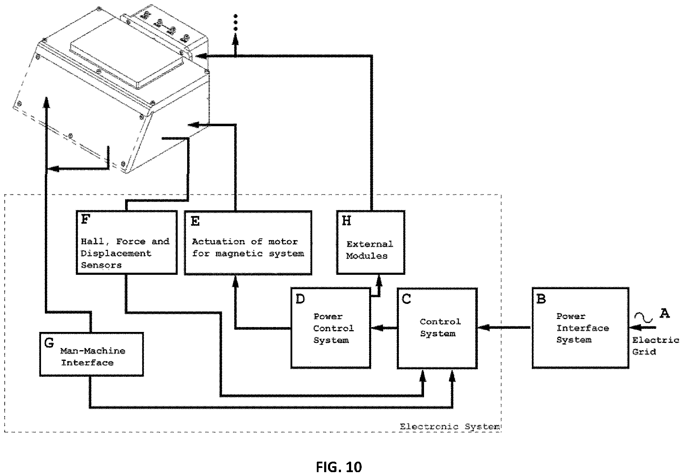

This group of parameters is transposed to the physical environment through an electronic control system, which is schemed in **FIG. 10**.

The system is powered through the electrical grid (**A**), where through an interface power system (**B**), external to the system, the voltage levels from electric grid (**A**) are transformed to appropriate values (230/115 Vac to Vdc continuous voltage up to 24 V). The system is then powered by appropriate voltage levels for the electronic control system, as well as all peripherals.

The electronic control system (**C**) performs the mechanical displacement system actuation (**E**), as well as the magnetic or electromagnetic actuation of said system actuation (**E**) and any extra module (H). Thus, it is possible to control the amplitude and the shape of the generated magnetic field produced by the electromagnets, in the case of being used, varying the electrical current that makes the electromagnets actuate, through a power electronic control system (**D**).

The system is based in a closed loop control, where the shape of the generated field is measured by a group of Hall Effect, force and displacement (**F**) sensors, installed preferably in the magnetic table (**5**), with a placement that allows analysing the shape of the field in completely magnetic table area. This provides the power control system with enough data to run the generated magnetic field as desired.

In the described system, it is possible to assemble at least two types of tissue culture that may vary within themselves according to selected user parameters such as displacement and frequency. The system allows enables magnetic field variations on magnetostrictive and magnetoelectric scaffolds, stretching forces in fibrous scaffolds and current stimuli in fibrous scaffolds embedded with conductive biocompatible particles, by linear displacement of permanent magnets or controlling the current frequency within electromagnets in table (**5**) or (**15**), accordingly to each type of stimuli configuration (either Module II or III).

This control is performed by the control unit (**C**), which runs the motor driver circuits employed, any motor may be employed as long as a closed loop control is available and temperatures are stable below cellular culture tolerances.

As a preferable embodiment of the present invention is the main target of this system, the cellular culture, the experiences can have a duration of several weeks. Thus, the system allows an autonomous operation during the entire period, permitting to define many groups of operating cycles, in a way that allows reproducing the stimuli present in the natural environment of the cells under growth.

The control system communicates with the man-machine interface unit (**G**) composed by a TFT or LCD or any other method which may present the culture parameters (**2**) and a capacitive or any other type of waterproof keyboard (**3**), interacting with the user and allowing the device configuration.

Despite of the invention has been described in some detailed by way of illustration and examples for a matter of clarity and understanding, it will be evident that certain changes and modifications can be practiced within the scope of the attached claims of this description.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## Claims

1. A modular magnetically driven bioreactor for applying stimuli to cell culture scaffolds comprising:
at least one cellular culture plate (d1, d2);
a first module comprising a support enclosure (a) and a magnetically inert cover (c) arranged above the support enclosure (a) for supporting the cellular culture plate (d1, d2);
an actuator module (b1, b2) arranged below the magnetically inert cover (c);
wherein said first module is arranged for receiving said actuator module (b1, b2);
wherein the actuator (b1, b2) comprises:
a fixed platform, also referred as a fixed table,
a movable platform, also referred as a movable table;
wherein said movable platform, also referred as a movable table, comprises a plurality of magnets for applying a movable magnetic field to the cellular culture plate (d1, d2).

2. The modular magnetically driven bioreactor according to the previous claim wherein said actuator module is a second module comprising a magnetic field actuator.

3. The modular magnetically driven bioreactor according to the previous claim wherein the fixed platform is arranged below the movable platform which is movable to apply a movable magnetic field to the cellular culture plate (d1, d2).

4. The modular magnetically driven bioreactor according to claim 1 wherein said actuator module is a third module comprising a stretching actuator.

5. The modular magnetically driven bioreactor according to the previous claim wherein the fixed platform is arranged laterally in respect of the movable platform to which is movable to apply a movable magnetic field to the cellular culture plate (d1, d2) in order to stretch the cell culture scaffold.

6. The modular magnetically driven bioreactor according to any of the claims 2-3 wherein said second module comprises a magnetic field actuator arranged for generating a magnetic field, wherein said magnetic field actuator is placed below the magnetically inert cover and comprises at least two platforms, a first platform being fixed to the first module and a second platform arranged to be moveable by a motor and wherein said second platform comprises an equally spaced matrix table of magnets.

7. The modular magnetically driven bioreactor according to any of the claims 4-5 wherein said third module comprises a stretching actuator arranged for generating electro-stretching stimuli, comprising a lower mechanical apparatus comprising at least two platform rows of magnets, said lower mechanical apparatus being arranged to change the distance between said rows; and holding clamps arranged for withstanding magnets to follow the magnetic force provided and thus stretching a cellular scaffold being held on the culture plate.

8. The modular magnetically driven bioreactor according to the claims 2 and 4 wherein the first module is arranged for receiving the second module comprising the magnetic field actuator and the third module comprising the stretching actuator, in particular the modular magnetically driven bioreactor comprising both the second module and the third module as interchangeable parts of a kit.

9. The modular magnetically driven bioreactor according to any of the previous claims further comprising an electrical impulses module, preferably the electrical impulses module being arranged as an external box-like structure to be coupled to the back of the first module for applying electrical contact, said electrical impulses module comprises an electronically controlled circuit board and corresponding connectors to receive power from an electrical grid and provide communication from the bioreactor.

10. The modular magnetically driven bioreactor according to any of the previous claims wherein said second module comprises a magnetic field actuator arranged for generating magnetic field, said magnetic field actuator is placed below the magnetically inert cover and comprises a motion mechanism in ball-screw assembly and at least two platforms, a first platform being fixed by screws and/or narrow fitting to the first module and a second platform arranged to be moveable alongside a screw axis actuated by a motor and said second platform comprises an equally spaced matrix of magnets.

11. The modular magnetically driven bioreactor according to any of the previous claims wherein the cellular culture plate comprises 24 or 48 wells and contains the scaffolds featuring magnetic properties, provided that the second module comprising the magnetic field actuator is the actuator.

12. The modular magnetically driven bioreactor according to any of the previous claims wherein the cellular culture plate comprises scaffold support between two scaffold clamps arranged on opposite sides, two contact plates attached to the scaffold support, and wirings, provided that the third module comprising the stretching actuator is the actuator.

13. The modular magnetically driven bioreactor according to the previous claim wherein the scaffold support comprises magnetostrictive and magnetoelectric elements, particularly magnetostrictive materials selected from cobalt ferrites, nickel ferrites and Terfenol-D, coated with piezoelectric or non-piezoelectric polymers or magnetoelectric materials or fibrous seeded with skeletal muscle cells.

14. The modular magnetically driven bioreactor according to the previous claim wherein the piezoelectric polymer is selected from poly(vinylidene fluoride) or polylactic acid and the non-piezoelectric polymer is selected from Poly(vinylidene fluoride) in the alpha phase or poly(lactic-co-glycolic acid).

15. The modular magnetically driven bioreactor according to the previous claim wherein the piezoelectric polymer is embedded with conductive particles selected from graphene, carbon nanotubes or ionic liquids.
